# EUROPÄISCHE PATENTANMELDUNG

(11) **EP 1 398 019 A1**
(43) Veröffentlichungstag der Anmeldung: **17.03.2004**
(21) Anmeldenummer: 02292247.0
(22) Anmeldetag: 13.09.2002
(51) Int. Cl.: A61K 7/00, A61K 7/48, A61K 38/39

(54) **Verfahren zum Schutz und zur Modulation von Dermal Epidermal Junctions**

(71) Anmelder: Cognis France S.A., 31360 Saint-Martory (FR)
(72) Erfinder: Pauly, Gilles, 54000 Nancy (FR); Jeanmaire, Christine, 54000 Nancy (FR)
(74) Vertreter: Cabinet HERRBURGER

(57) **Zusammenfassung**

Vorgeschlagen wird ein Verfahren zur kosmetischen Behandlung zur Verbesserung und/oder zum Schutz der Dermal-Epidermal-Junctions der Haut, Kopfhaut und Schleimhaut, dadurch gekennzeichnet, dass man eine Zubereitung, enthaltend mindestens eine Substanz, welche eine Modulation von Plectin/HD1 und/oder Entactin/Nidogen und/oder Perlecan bewirkt, topisch aufträgt.

Des Weiteren wird die Verwendung einer Substanz, welche eine Modulation von Plectin/HD1 und/oder Entactin/Nidogen und/oder Perlecan bewirkt, zur Herstellung kosmetischer Mittel zur Verbesserung der Dermal Epidermal Junctions der Haut, Kopfhaut und Schleimhaut und die Verwendung dieser Substanz zur Herstellung kosmetischer Mittel zum Schutz gegen Hautalterung, oxidativen Stress und die Verwendung der Substanz zur Herstellung kosmetischer Mittel zum Schutz gegen schädigende Einflüsse durch Umweltgifte und UV-Strahlung vorgeschlagen.

## Beschreibung

Die Erfindung befindet sich auf dem Gebiet der kosmetischen Zubereitungen und betrifft ein kosmetisches Verfahren zur Verbesserung und/oder zum Schutzder Dermal-Epidermal Junctions der Haut, Kopfhaut und Schleimhaut und zum Schutz der menschlichen Haut gegen die Alterung, oxidativen Stress und gegen schädigende Einflüsse durch Umweltgifte und UV-Strahlung. Des weiteren betrifft die Erfindung die Verwendung einer Substanz, welche eine Modulation von Plectin/HD1 und/oder Entactin/Nidogen und/oder Perlecan bewirkt, zur Herstellung kosmetischer Mittel zur Verbesserung und/oder zum Schutz der Dermal Epidermal Junctions.

### Stand der Technik

Die Basalmembran ist eine verbindende Zellstruktur zwischen morphologisch unterschiedlichen Geweben. In der Haut befindet sie sich im wesentlichen als Blutgefäße umschließendes Gewebe und zwischen Dermis und Epidermis. Die letztgenannte Region trennt die Epidermis und ihre Anhanggebilde von der Dermis und wird entsprechend als Dermal-Epidermal-Junction (DEJ) bezeichnet. Die DEJ besitzt eine komplexe Struktur bestehend aus Hemidesmosomen, intermediären Filamenten, verankernden Filamenten, Lamina densa und verankernden Fibrillen. Zu den darin hauptsächlich vorkommenden biochemischen Komponenten zählen Laminin-5 in der Lamina lucida; die Antigene AgBP 230 und AgPB180 sowie Plectin/HD1 in Hemidesmosomen; Entactin/Nidogen und das Proteoglykan Perlecan in der Lamina lucida und Lamina densa; Collagen Typ IV in der Lamina densa und das Proteoglykan Collagen Typ VII als Bestandteil der verankernden Fibrillen in der sub-Lamina densa. Diese Komponenten bilden ein interagierendes Netzwerk.

Die DEJ stellt das wichtigste Gebilde der Haut dar. Sie sorgt für die Verbindung zwischen Epidermis und darunterliegender Dermis und erhält die Integrität des epithelialen Gewebes durch Verankerung von Zellen mit der extrazellulären Matrix über die speziellen verbindenden Zellkomplexe der Hemi-Desmosomen, Filamente und Fibrillen.

Einerseits stellt sie einen Filter für den Fluß spezieller Moleküle dar, andererseits ermöglicht sie den Austausch von Informationen - beispielsweise über Wachstumsfaktoren - zwischen Keratinocyten und Dermis.

Zu den alterungsbedingten Änderungen der DEJ zählen die abnehmende Dicke der Junctions und die Reduktion von den aus basalen Keratinocyten bestehenden cytoplasmatischen Zellzotten in der Dermis. Die dadurch resultierende Abnahme der Oberfläche der DEJ führt zu einem reduzierten Widerstand des Gewebes, einer Abnahme der Hautstraffung und einer vermehrten Bildung von Falten. Weitere Alterungserscheinungen wie Verdopplung der Lamina densa, Alterung der verankernden Fibrillen oder Modifikationen von Zellkomponenten der DEJ führen ebenfalls dazu, dass die Verankerung über das epidermale System mit zunehmendem Alter eine Lockerung erfährt. Die bei der Alterung bedingte Abnahme von Typ VII Collagen erklärt man sich auch durch die Hydrolyse des Collagens über Metalloproteinasen. Typ VII Collagen scheint mit zunehmendem Alter weniger resistent gegen Proteasen zu sein.

Auch durch UV-Strahlung induzierte Schädigungen, die zu einer Reduktion des Gehaltes an Typ VII Collagen in den verankernden Fibrillen führen, resultieren in einer Lockerung der Bindung zwischen Dermis und Epidermis und einer dadurch bedingten Abnahme der Hautelastizität und Zunahme von Falten.

Zahlreiche Krankheiten, die die DEJ beeinträchtigen, führen zu einer Ausbildung von subepidermalen Umfangsvermehrungen. Sie zeigen als gemeinsames Merkmal eine verminderte Cohesion zwischen Dermis und Epidermis, die sich auf der Ebene der DEJ in einer Bildung von Einbuchtungen manifestiert.

Mit dem Ziel einer Verbesserung der Funktion der Dermal Epidermal Junctions wurden insbesondere Bestandteile der DEJ wie Lamin (FR 2813018, WO 97/48415), oder Kalinin (WO 92/ 17498) in kosmetischen und dermatologischen Formulierungen eingesetzt. Auch die Stimulation von Typ IV Collagen durch Magnesiumaspartat (WO 99/62481), Saponine (FR 2779058) oder Pflanzenextrakte (EP 668072) sowie die Stimulation von Typ VII Collagen durch Elaginsäure (WO 99/16415), Pflanzenextrakt der Potentilla erecta (WO 98/19664) oder Bertholletia Extrakt (US 6004568) wurden offenbart zur Herstellung von kosmetischen Zubereitungen gegen Alterung, Faltenbildung und zur Straffung der Haut.

Dennoch besteht weiterer Bedarf an einem effektiven Schutz der Haut gegen umweltbedingte Alterungseinflüsse. Die Aufgabe der vorliegenden Patentanmeldung hat daher darin bestanden, neue Mechanismen zur Verbesserung der Dermal-Epidermal Junctions der Haut, Kopfhaut und Schleimhaut zu finden, die zu einer Verzögerung der Hautalterung und zu einem Schutz der Haut, Kopfhaut und Schleimhaut gegen Umwelteinflüsse, oxidativen Stress, toxische Substanzen oder UV-Strahlung beitragen und somit effektiv in kosmetischen Zubereitungen für die topische Anwendung genutzt werden können.

### Beschreibung der Erfindung

Gegenstand der Erfindung sind Verfahren zur kosmetischen Behandlung zur Verbesserung und/oder zum Schutz der Dermal-Epidermal Junctions der Haut, Kopfhaut und Schleimhaut, dadurch gekennzeichnet, dass man eine Zubereitung, enthaltend mindestens eine Substanz, welche eine Modulation von Plectin/HD1 und/oder Entactin/Nidogen und/oder Perlecan bewirkt, topisch aufträgt.

Weitere Gegenstände der Erfindung sind die Verwendung einer Substanz, welche eine Modulation von Plectin/HD1 und/oder Entactin/Nidogen und/oder Perlecan bewirkt, zur Herstellung kosmetischer Mittel zur Verbesserung und/oder zum Schutz der Dermal-Epidermal Junctions der Haut, Kopfhaut und Schleimhaut, die Verwendung dieser Substanz zur Herstellung kosmetischer Mittel zum Schutz gegen Hautalterung und die Verwendung der Substanz zur Herstellung kosmetischer Mittel zum Schutz gegen oxidativen Stress, schädigende Einflüsse durch Umweltgifte und UV-Strahlung.

Überraschenderweise wurde gefunden, dass die Modulation von Molekülen wie Plectin/HDl, Entactin/Nidogen und/oder Perlecan zu einer Erhaltung und Verbesserung der Dermal-Epidermal-Junctions der Haut, Kopfhaut und Schleimhaut führt. Die Funktion der DEJ ist eine essentielle Voraussetzung nicht nur für die Gesundheit, sondern auch für die Kosmetik. Sie sorgt für einen guten Zusammenhalt zwischen Epidermis und den darunter liegenden Geweben der Dermis, erhält somit die Elastizität und Straffheit der Haut und ermöglicht die Verhinderung von Faltenbildung. Darüber hinaus wird über die DEJ einerseits die gute Versorgung der Haut durch die Passage von lebensnotwendigen Molekülen zwischen Epidermis und Dermis gewährleistet, andererseits ein Schutz vor dem Eindringen schädigender Moleküle in tiefere Hautschichten geboten.

Die Modulation von Plectin/HD1, Entactin/Nidogen und/oder Perlecan über die topische Applikation einer Zubereitung, die diese Substanzen stimuliert, hat nun gezeigt, dass auf diese Weise die Dermal Epidermal Junction resp. das gesamte komplexe Netzwerk der DEJ gestärkt werden kann. Dieses hat eine Straffung der Haut und Verminderung der Faltenbildung zur Folge. Durch die verbesserte Verankerung zwischen den Komponenten der DEJ und die damit verbundene erhöhte Stabilität und zunehmende Elastizität des Gewebes können Alterungserscheinungen, auch wenn sie durch UV-Strahlung verursacht sind, effektiv vorgebeugt werden.

Bedingt durch die Verbesserung der Molekülpassagefunktion wird der Austausch zwischen Keratinocyten und Dermis und die Ernährung der Haut optimiert, und somit nicht nur die Haut gegen Alterung, sondern auch gegen schädigende Einflüsse von UV-Strahlung und toxischen Umwelteinflüssen geschützt, da durch die verbesserte Versorgung auch die Abwehr gegen schädigende Moleküle gestärkt wird.

Die DEJ umgibt auf der Kopfhaut die Haarfollikel und sorgt auch hier für einen Schutz der Follikel, so dass eine Stärkung der DEJ in diesem Bereich zu einer Verbesserung der Haareigenschaften führt und insbesondere wirksam ist gegen Haarausfall oder Haarschädigungen.

Somit haben die topisch applizierten kosmetischen Mittel, die mindestens eine Substanz enthalten, welche die Modulation von Plectin/HD1 und/oder Entactin/Nidogen und/oder Perlecan bewirkt, einen vorbeugenden Effekt gegen Hautalterung und schädigende Einflüsse durch oxidativen Stress, Umweltgifte und UV-Strahlung auf Haut, Kopfhaut, Haare und Schleimhaut. Die Modulation der speziellen Moleküle führt jedoch neben der vorbeugenden Wirkung auch zu einer beschleunigten Regeneration der Haut, Kopfhaut und Schleimhaut nach einer bereits eingetretenen Schädigung.

Als Modulatoren haben sich Pflanzenextrakte, insbesondere der Extrakt aus Pisum sativum als geeignet erwiesen. Möglich ist jedoch auch eine Modulation von Plectin/HD1 und/oder Entactin/Nidogen und/oder Perlecan über die Gabe von niedrigmolekularen Peptiden, die zum Aufbau der DEJ-Komponenten benötigt werden und eine ähnliche Sequenz aufweisen wie Bestandteile von Plectin/HD1 und/oder Entactin/Nidogen und/oder Perlecan.

Ebenfalls beobachtet wurde eine Modulation der Moleküle über mindestens eine Substanz, die ausgewählt ist aus der Gruppe, die gebildet wird von Mannitol, Cyclodextrin, Hefeextrakt, Dinatriumsuccinat. Insbesondere die Kombination dieser Bestandteile trägt zu einer vorteilhaften Wirkung auf die Dermal-Epidermal Junctions bei.

Da die DEJ für den Abbau durch eine Vielzahl unterschiedlicher Proteasen sehr empfänglich ist, trägt die Verwendung von Pflanzenextrakten, Peptiden und anderen Wirkstoffen mit Anti-Protease Aktivität entscheidend zum Erhalt der Struktur bei. Wirkstoffe mit Anti-Protease Aktivität können in Kombination mit Substanzen, welche die Modulation von Plectin/HD1 und/oder Entactin/Nidogen und/oder Perlecan bewirken, zu einer überdurchschnittlichen Wirkungssteigerung beitragen.

Neben diesen Substanzen oder Pflanzenextrakten können die kosmetischen Mittel außerdem UV-Lichtschutzfaktoren und/oder Antioxidantien enthalten. Die Kombination aus Substanzen, die eine Modulation von Plectin/HDI und/oder Entactin/Nidogen und/oder Perlecan bewirken mit UV-Lichtschutzfaktoren und/oder Antioxidantien führt durch die unterschiedlichen Mechanismen zu einer synergistischen Wirkungsweise und bietet einen hervorragenden Schutz gegen schädigende Einflüsse und Hautalterung durch UV-Lichteinwirkung.

### Plectin/HD1

Plectin und HD1 sind Synonyme für das selbe Molekül. Es ist in den Hemi-Desmosomen lokalisiert, weist eine Molekülmasse von ca. 500 Dalton auf und dient der Verankerung von Proteinen des Cytoskellets wie Keratin, Vimentin oder Proteinen der Microtubuli.

### Entactin/Nidogen

Auch Entactin und Nidogen sind unterschiedliche Begriffe für das selbe Molekül. Es stellt ein Glycoprotein mit einer Molekülmasse von ungefähr 150 Dalton dar, das aus zwei terminalen globulären Regionen, die durch eine langkettige Struktur miteinander verbunden sind, besteht. Dieses Glycoprotein ist maßgeblich für die strukturelle Stabilität der DEJ verantwortlich, dadurch dass es eine Verbindung zwischen Laminin und Typ IV Collagen herstellt und zur Verankerung einer Vielzahl weiterer Komponenten wie Fibulin oder Perlecan beiträgt.

### Perlecan

Alle Basalmembranen enthalten Proteoglycane. Das hauptsächlich in der DEJ vorkommende Proteoglycan ist Perlecan, ein Heparansulfat, das von dermalen Fibroblasten synthetisiert wird. Perlecan besteht aus einem großen Proteinkern und drei Heparansulfatketten. Seine Aufgabe ist es unter anderem eine Bindung zwischen Laminin-6 und Nidogen zu stabilisieren. Neben den verankernden Funktionen binden Heparansulfat-Proteoglykane auch diffundierende Moleküle wie Enzyme und Wachstumsfaktoren und haben möglicherweise dadurch ebenfalls einen Einfluss auf das Verhalten und die Eigenschaften von Zellen.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
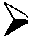 3-Benzylidencampher bzw. 3-Benzylidennorcampher und dessen Derivate, z.B. 3-(4-Methylbenzyliden)campher;
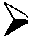 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
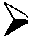 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
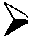 Ester der Salicylsäure, vorzugsweise Salicylsäure-2-ethylhexylester, Salicylsäure-4-isopropylbenzylester, Salicylsäurehomomenthylester;
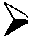 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
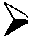 Ester der Benzalmalonsäure, vorzugsweise 4-Methoxybenzmalonsäuredi-2-ethylhexylester;
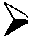 Triazinderivate, wie z.B. 2,4,6-Trianilino-(p-carbo-2'-ethyl-1'-hexyloxy)-1,3,5-triazin und Octyl Triazon, oder Dioctyl Butamido Triazone (Uvasorb® HEB);
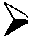 Propan-1,3-dione, wie z.B. 1-(4-tert.Butylphenyl)-3-(4'methoxyphenyl)propan-1,3-dion;
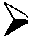 Ketotricyclo(5.2.1.0)decan-Derivate.

Als wasserlösliche Substanzen kommen in Frage:
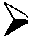 2-Phenylbenzimidazol-5-sulfonsäure und deren Alkali-, Erdalkali-, Ammonium-, Alkylammonium-, Alkanolammonium- und Glucammoniumsalze;
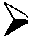 Sulfonsäurederivate von Benzophenonen, vorzugsweise 2-Hydroxy-4-methoxybenzophenon-5-sulfonsäure und ihre Salze;
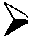 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'methoxydibenzoylmethan (Parsol® 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄ Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

## Patentansprüche

1. Verfahren zur kosmetischen Behandlung zur Verbesserung und/oder zum Schutz der Dermal-Epidermal-Junctions der Haut, Kopfhaut und Schleimhaut, **dadurch gekennzeichnet, dass** man eine Zubereitung, enthaltend mindestens eine Substanz, welche eine Modulation von Plectin/HD1 und/oder Entactin/Nidogen und/oder Perlecan bewirkt, topisch aufträgt.

2. Verwendung einer Substanz, welche eine Modulation von Plectin/HD1 und/oder Entactin/Nidogen und/oder Perlecan bewirkt, zur Herstellung kosmetischer Mittel zur Verbesserung und/oder zum Schutz der Dermal-Epidermal-Junctions der Haut, Kopfhaut und Schleimhaut.

3. Verwendung einer Substanz, welche eine Modulation von Plectin/HD1 und/oder Entactin/Nidogen und/oder Perlecan bewirkt, zur Herstellung kosmetischer Mittel zum Schutz der Haut gegen Alterung.

4. Verwendung einer Substanz, welche eine Modulation von Plectin/HD1 und/oder Entactin/Nidogen und/oder Perlecan bewirkt, zur Herstellung kosmetischer Mittel zum Schutz der Haut, Kopfhaut und Schleimhaut gegen toxische Umwelteinflüsse.

5. Verwendung einer Substanz, welche eine Modulation von Plectin/HD1 und/oder Entactin/Nidogen und/oder Perlecan bewirkt, zur Herstellung kosmetischer Mittel zum Schutz der Haut, Kopfhaut und Schleimhaut gegen UV-Lichteinwirkung.

6. Verwendung einer Substanz, welche eine Modulation von Plectin/HD1 und/oder Entactin/Nidogen und/oder Perlecan bewirkt, zur Herstellung kosmetischer Mittel gegen oxidativen Stress.

7. Verwendung einer Substanz, welche eine Modulation von Plectin/HD1 und/oder Entactin/Nidogen und/oder Perlecan bewirkt, zur Herstellung kosmetischer Mittel gegen Haarausfall und zur Verbesserung der Haareigenschaften.

8. Verwendung gemäß mindestens einem der Ansprüche 2 bis 7, **dadurch gekennzeichnet, dass** die Substanz ein Pflanzenextrakt ist, welcher eine Modulation von Plectin/HD1 und/oder Entactin/Nidogen und/oder Perlecan bewirkt.

9. Verwendung gemäß Anspruch 8, **dadurch gekennzeichnet, dass** der Extrakt aus der Pflanze Pisum sativum gewonnen ist.

10. Verwendung gemäß mindestens einem der Ansprüche 2 bis 7, **dadurch gekennzeichnet, dass** die Substanz ausgewählt ist aus der Gruppe, die gebildet wird von Mannitol, Cyclodextrin, Hefeextrakt, Dinatriumsuccinat, niedrigmolekularen Peptiden.

11. Kosmetische Zubereitungen, enthaltend mindestens eine Substanz, welche eine Modulation von Plectin/HD1 und/oder Entactin/Nidogen und/oder Perlecan bewirkt, und UV-Lichtschutzfaktoren und/oder Antioxidantien.
